# EUROPEAN PATENT APPLICATION

(11) **EP 1 123 708 A1**
(43) Date of publication of application: **16.08.2001**
(21) Application number: 99940662.2
(22) Date of filing: 03.09.1999
(51) Int. Cl.: A61K 35/78, A61K 31/19, A61K 31/215, A23L 3/3472

(54) **TEAR GRASS-DERIVED ANTIBACTERIAL AGENT AND PROCESS FOR PRODUCING THE SAME**

(30) Priority: 04.09.1998 JP 26582398; 11.01.1999 JP 357799; 30.08.1999 JP 24266499
(71) Applicant: Freund Industrial Co., Ltd., Tokyo 169-0075 (JP)
(72) Inventor: SHOBU, Tomoyuki, R & D Laboratories, Hamamatsu-shi, Sizuoka 431-2103 (JP); IMAMURA, Natsuko, R & D Laboratories, Hamamatsu-shi, Sizuoka 431-2103 (JP); SAITO, Yoshihito, R & D Laboratories, Hamamatsu-shi, Sizuoka 431-2103 (JP); HOSHINO, Satomi, R & D Laboratories, Hamamatsu-shi, Sizuoka 431-2103 (JP)
(74) Representative: Ziebig, Marlene, Dr. Dipl.-Chem.
(86) International application number: JP9904803
(87) International publication number: WO0013697

(57) **Abstract**

The object of the present invention is to provide an antibacterial substance derived from Job's-tears having an improved antibacterial effect. It was found that the antibacterial effect of grass tear or an extract therefrom is improved by heat-processing. It was further found that, among the components of the Job's-tears having an improved antibacterial effect, a component having a particularly strong antibacterial effect is contained in lipid components thereof.

## Description

### Technical Field

The present invention relates to a natural antibacterial agent having an excellent antibacterial effect and a method for producing the same. It relates particularly to a Job's-tears (Coix lacryma-jobi Linne var. ma-yuen Stapf (Gramineae))-derived antibacterial agent having an improved antibacterial effect and a method for producing the same. Furthermore, the present invention relates to an antibacterial composition containing a Job's-tears-derived antibacterial component.

### Prior Art

As antibacterial agents, chemosynthesis products such as sorbic acid and benzoic acid have previously been used. Since chemosynthesis products have an excellent antibacterial effects and can be produced at low cost, these are used in various fields. In recent years, however, chemosynthesis products have been given a wide berth because of concerns about safety, and natural components are growing in demand.

There are many types of natural antibacterial agents including various antibacterial agents such as protamine and polylysine, which are already on market. One of the commercialized antibacterial agent is a Job's-tears-derived antibacterial agent (Japanese Patent Application Laid-Open (kokai) No. 51-67721). For this antibacterial agent, powdered Job's-tears or an extract from Job's-tears is used as a natural preservative agent. Used as a material for rice crackers or other confectioneries, Job's-tears is a much safer crude drug, which is described as coix seed and powdered coix seed in *the Japanese Pharmacopoeia* 13^{th} Edition.

Nevertheless, a Job's-tears-derived preservative agent produced by the method described in the above-mentioned publication of unexamined application, is deficient in that it has insufficient antibacterial effect and that this antibacterial effect varies depending on the locality or variety of Job's-tears used as a material.

An object of the present invention is to overcome the above-described problems regarding the conventional Job's-tears-derived antibacterial or preservative agent, and to provide a Job's-tears-derived antibacterial or preservative agent having a stable and strong antibacterial effect regardless of the locality or variety of Job's-tears material.

Another object of the present invention is to provide both a method for improving the antibacterial effect of components contained in Job's-tears and a method for separating and obtaining a component showing an excellent antibacterial effect from Job's-tears.

### Disclosure of the Invention

As a result of a thorough study to develop a Job's-tears-derived antibacterial agent having a stronger and more stable antibacterial effect, the present inventors have found that the antibacterial effect of Job's-tears and an extract therefrom is improved by heat-processing. The present inventors have further found that, among the components of the Job's-tears having such improved antibacterial effect, a component having a particularly strong antibacterial effect is contained in lipid components thereof, thereby completing the present invention. Furthermore, the present inventors have also found that the antibacterial activity of a Job's-tears-derived antibacterial substance is promoted by certain types of substance group, thereby completing the present invention.

The present invention comprises each of the following inventions:
(1) An antibacterial agent which comprises heat-processed Job's-tears.
(2) An antibacterial agent which comprises an extract from heat-processed Job's-tears or a heat-processed product of an extract from Job's-tears.
(3) An antibacterial agent which comprises lipid components obtained from, an extract from heat-processed Job's-tears or a heat-processed product of an extract from Job's-tears.
(4) The antibacterial agent according to (3) above, wherein the above lipid components are simple lipid components.
(5) The antibacterial agent according to (3) or (4) above, wherein the above lipid components, which are obtained from the above extract from heat-processed Job's-tears and/or the product of the heat-processed extract from Job's-tears, are lipid components corresponding to a fraction of preparative high performance liquid chromatography at retention time 20 to 40 min., conducted under the following conditions:

| Conditions on preparative HPLC | |
|---|---|
| Preparative column | Inertsil PREP-ODS (reversed-phase partition system, GL |
| | Sciences Inc.) |
| Column size | 20.0mm × 250mm |
| Eluant | Liquid A (acetonitrile and water (7:3)) |
| | Liquid B (acetonitrile) |
| Gradient | 0 to 50 min. A |
| | 50 to 70 min. A→B linear gradient |
| | 70 to 115 min. B |
| Flow rate | 10.0ml/min. |
| Detection | UV 195nm |
| Column temperature | 40°C |

(6) An antibacterial agent, which comprises:
at least one Job's-tears-derived component selected from a group which consists of a component consisting of an antibacterial agent described in any one of (1) to (5) above, and another component consisting of an extract or an enzymolysis extract from unheated Job's-tears; and,
at least one component selected from a group which consists of amino acids, amino acid salts, carboxylic acids, carboxylic acid salts, inorganic acids, inorganic acid salts, fatty acid esters, ethanol, protamine and lysozyme.

(7) An antibacterial agent, which comprises:
at least one Job's-tears-derived component selected from a group which consists of a component consisting of an antibacterial agent described in any one of (1) to (5) above, and another component consisting of powders or an extract obtained from unheated Job's-tears; and,
at least one component selected from a group consisting of glycine, alanine,
glutamic acid, asparatic acid, acetic acid, lactic acid, succinic acid, citric acid, sorbic acid, fumaric acid, malic acid, tartaric acid, gluconic acid, glutaric acid, adipic acid,
propionic acid, benzoic acid, cinnamic acid, hydrochloric acid, pyrophosphoric acid, phosphoric acid, polyphosphoric acid, phytic acid or phytic acid salts, gluconodeltalactone, ethanol, glycerine fatty acid ester, sucrose fatty acid ester, sorbitan fatty
acid ester, propylene glycol fatty acid ester, protamine and lysozyme.

(8) A method for antibacterial processing of a target which comprises using a component contained in an antibacterial agent described in any one of (1) to (5) above; or using at least one Job's-tears-derived component selected from a group consisting of the component contained in the antibacterial agent, and powders or an extract obtained from unheated Job's-tears, in combination with at least one component selected from a group consisting of amino acids, amino acid salts, carboxylic acids, carboxylic acid salts, inorganic acids, inorganic acid salts, fatty acid esters, ethanol, protamine and lysozyme.
(9) A method for producing an antibacterial component contained in an antibacterial agent, wherein Job's-tears and/or an extract from Job's-tears are/is heat-processed at 50 to 250°C.
(10) A method for producing an antibacterial component for an antibacterial agent, which comprises heat-processing Job's-tears and/or an extract from Job's-tears at 50
to 250°C, and separating lipid components from the heat-processed Job's-tears and/or extract from Job's-tears.
(11) A method for producing an antibacterial component for an antibacterial agent, which comprises obtaining lipid components from a heat-processed powdered Job's-tears or a product of an extract from unheated Job's-tears, wherein said lipid components correspond to a fraction of preparative high performance liquid chromatography at retention time 20 to 40 min., conducted under the following conditions:

| Conditions on preparative HPLC | |
|---|---|
| Preparative column | Inertsil PREP-ODS (reversed-phase partition system, GL Sciences Inc.) |
| Column size | 20.0mm × 250mm |
| Eluant | Liquid A (acetonitrile and water (7:3)) |
| | Liquid B (acetonitrile) |
| Gradient | 0 to 50 min. A |
| | 50 to 70 min. A→B linear gradient |
| | 70 to 115 min. B |
| Flow rate | 10.0ml/min. |
| Detection | UV 195nm |
| Column temperature | 40° C |

### Brief Description of the Drawings

Figure 1 is a chromatogram showing the result of HPLC on a Job's-tears-derived antibacterial substance (production district A) in Example 2.

Figure 2 is a chromatogram showing the result of HPLC on another Job's-tears-derived antibacterial substance (production district B) in Example 2.

Figure 3 is a chromatogram showing the result of HPLC on a Job's-tears-derived antibacterial substance (production district A) in Control example 2.

Figure 4 is a chromatogram showing the result of HPLC on another Job's-tears-derived antibacterial component (production district B) in Control example 2.

Figure 5 is a chromatogram on total lipids having a Job's-tears-derived antibacterial effect, which was obtained at the time of preparative HPLC measurement.

Figure 6 is a chromatogram showing the result of analytical HPLC on total lipids having a Job's-tears-derived antibacterial effect.

Figure 7 is a chromatogram showing the result of analytical HPLC on lipids corresponding to fraction 4 in total lipids having a Job's-tears-derived antibacterial effect.

Figure 8 is a chromatogram showing the result of preparative HPLC on total lipids contained in unheated Job's-tears.

Figure 9 is a chromatogram showing the result of analytical HPLC on total lipids contained in unheated Job's-tears.

Figure 10 is a chromatogram showing the result of analytical HPLC on lipids corresponding to fraction 4 in total lipids contained in unheated Job's-tears.

### The Best Mode for Carrying out the Invention

With regard to Job's-tears material used for the production of a Job's-tears-derived antibacterial agent or substance of the present invention, the production district and variety are not particularly limited, and the form is also not particularly limited. However, taking heat-processing efficiency into consideration, powdered Job's-tears is preferable.

Examples of an extract from Job's-tears include, but are not limited to, an extract obtained by extraction from Job's-tears, particularly from heat-processed Job's-tears using a hydrophilic organic solvent such as ethanol and then removal of the solvent from the extract, or a pressed liquid obtained from heat-processed Job's-tears using a compressor.

The heat-processing of Job's-tears and an extract from Job's-tears in the production method of the present invention means that Job's-tears material or an extract from Job's-tears is heat-processed at appropriate temperature in order to improve an antibacterial effect thereof.

Heat-processing temperature is preferably 50 to 250°C, and more preferably 100 to 200°C. Neither equipment nor methods applied for heat-processing are particularly limited.

The optimum heat-processing time is not determined, but depends on the performance of the equipment, heat-processing temperature, processing conditions such as the presence or absence of stirring during heat-processing, the variety of Job's-tears material, or the type of extract from Job's-tears. So, for each of these conditions, an optimum heat-processing time is established.

The precise mechanism for the improvement of antibacterial effect of Job's-tears or an extract from Job's-tears by heat-processing in the present invention is left for further study. However, from the results of high performance liquid chromatography indicating that different patterns of chromatograms are obtained by the presence or absence of heat-processing, it is clear that a certain change is produced by heat-processing. It has been confirmed that a component in total lipids contained in a Job's-tears extract is involved in the improvement of antibacterial effect, above all, simple lipid components have a strong antibacterial effect.

The Job's-tears-derived antibacterial agent or substance of the present invention can be obtained by heat-processing an extract from Job's-tears or by extracting from heat-processed Job's-tears, but methods for obtaining an extract from Job's-tears are not limited thereto. Methods for obtaining the extract include solvent extraction, the combined use of solvent extraction and enzymolysis, supercritical-fluid extraction, a compression method and the like, but are not limited thereto. The obtained extract may be converted into a concentrate containing 10 weight % of Job's-tears-derived antibacterial components by removing solvent therefrom, or the extract may be used as is.

Furthermore, processes such as the removal of any given components by solvent fraction, chromatography or the like, or the separation of lipid components having a strong antibacterial effect may be performed.

Fractions which contain lipid components having a strong antibacterial effect may be obtained from a heat-processed Job's-tears extract by a solvent fraction, chromatography or the like.

Methods for obtaining lipid components include the use of a mixed solvent of methanol and chloroform, a solvent extraction using ether or the like, a supercritical-fluid extraction etc., but are not limited thereto. Examples of chromatography include, but are not limited to, a preparative high performance liquid chromatography, wherein octadecyl-binding silica gel acts as a stationary phase.

The antibacterial effect of the Job's-tears-derived antibacterial agent or substance of the present invention can be improved by the combined use thereof with at least one component selected from a group which consists of amino acids, amino acid salts, carboxylic acids, carboxylic acid salts, inorganic acids, inorganic acid salts, fatty acid esters, ethanol, protamine and lysozyme.

Concrete examples of these components include glycine, alanine, glutamic acid, asparatic acid, acetic acid, lactic acid, succinic acid, citric acid, sorbic acid, fumaric acid, malic acid, tartaric acid, gluconic acid, glutaric acid, adipic acid, propionic acid, benzoic acid, cinnamic acid, hydrochloric acid, pyrophosphoric acid, phosphoric acid, polyphosphoric acid, phytic acid or phytic acid salts, gluconodelta-lactone, ethanol, glycerine fatty acid ester, sucrose fatty acid ester, sorbitan fatty acid ester, propylene glycol fatty acid ester, protamine, lysozyme etc.

Methods for improving the antibacterial effect of the Job's-tears-derived antibacterial agent or substance of the present invention by combined use with the above-listed components are not particularly limited, but all that is needed is the simple mixing of the above components and the Job's-tears-derived antibacterial *agent or* substance at an appropriate ratio.

For ease of handling, the Job's-tears-derived antibacterial agent or substance may be converted into solution or powder material before mixing with the above-listed components. When used as solution, it is preferable that the Job's-tears-derived antibacterial agent or substance is dissolved, emulsified or dispersed in water, ethanol or a mixture thereof, followed by the addition of the above-listed components.

On the other hand, when used as a powder material, it is preferable that dextrin, corn starch, lactose, pregelatinized starch, crystalline cellulose or the like is supported by the Job's-tears-derived antibacterial agent or substance, followed by the addition of the above-listed components.

Target products to which the antibacterial agent or substance of the present invention is added are not limited, but the examples include a wide range of foods such as fish pastry products, ham, sausage, delicatessen etc.

The antibacterial agent or substance of the present invention in itself may be added to target products such as food, or may previously be mixed with the above-listed components before addition to the target products. Otherwise, both the antibacterial agent or substance of the present invention and the components used in combination may independently be added to the target products.

In the present invention, 0.1 to 10 weight % of the antibacterial agent or substance is added to target products such as food.

The present invention is further described in the following examples. The example is provided for illustrative purposes only, and is not intended to limit the scope of the invention.

### Examples

### Example 1:

### Antibacterial activity of heat-processed Job's-tears

A powdered Job's-tears was heat-processed at 150°C for 3 hours in a forced convection constant temperature oven. Using *Bacillus subtilis* as a test bacteria, the heat-processed powdered Job's-tears was tested according to the following method, thereby determining MIC (minimum inhibitory concentration). The result is shown in Table 1.

### MIC measurement

MIC was measured according to the method of the Japanese Society of Chemotherapy. A standard agar medium was used, and the heat-processed powdered Job's-tears was added thereto, so that the concentrations of the powder would be 0.0005 to 1.6%. Then, test bacteria (3.5 × 10³/ml) was applied into the medium. The test bacteria was cultured at 35°C, and then MIC, i.e., a minimum concentration wherein the growth of bacteria was inhibited by a heat-processed powdered Job's-tears was determined.

### Example 2:

### Heating process

Two types of powdered Job's-tears were prepared, whose production districts differed (powders of Chinese origin: hereinafter referred to as "production district A", and mixed powders of both Chinese and Thai origins: hereinafter referred to as "production district B"). Each of the two types of powdered Job's-tears was heat-processed at 150°C for 1.5 hours in a forced convection constant temperature oven.

### Extraction and concentration process

1,500g of hydrous ethanol (concentration: 45 weight %) was added to 100g each of the above-obtained heat-processed products. While stirring, the mixture was extracted at room temperature (25° C) for 3 hours. After extraction, the whole extract was filtrated to remove solid elements, thereby obtaining an extract. Using a rotary evaporator, solvent was removed from the obtained extract to obtain a Job's-tears-derived antibacterial substance (production district A: 4.3g, production district B: 2.5g).

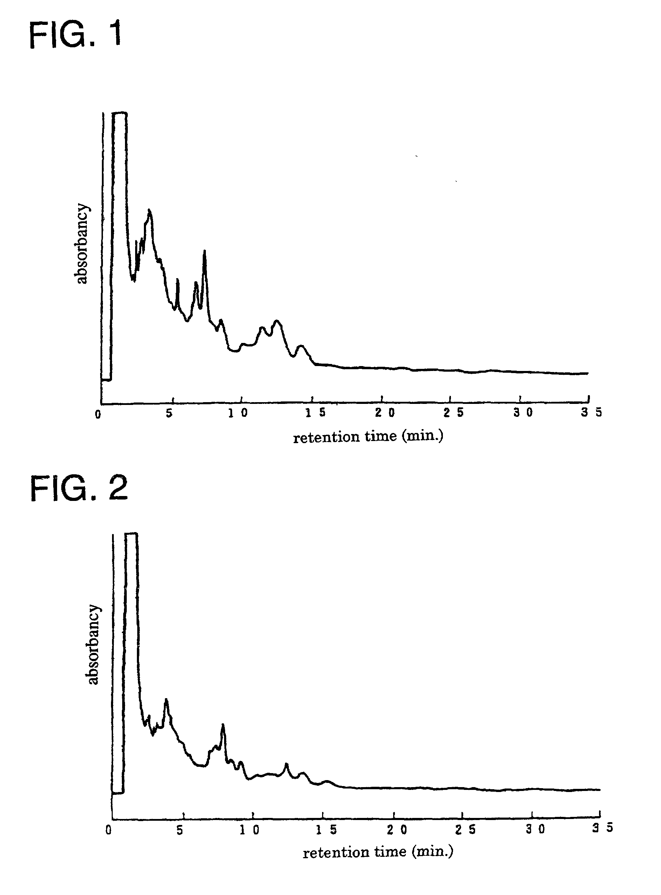

By performing high performance liquid chromatography for each of the obtained two kinds of antibacterial substances under the following conditions, chromatograms were obtained. The chromatogram of the substance of production district A is shown in Figure 1, and that of production district B is shown in Figure 2. Using *Bacillus subtilis* as a test bacteria, each of the obtained heat-processed Job's-tears substances was tested according to the same method as in Example 1, thereby determining MIC (minimum inhibitory concentration). The results are shown in Table 1.

| Conditions on high performance liquid chromatography | |
|---|---|
| Column | Inertsil ODS-3 (GL Sciences Inc.) |
| Column size | 4.6mm × 150mm |
| Column temperature | 40°C |
| Eluant | Acetonitrile and Water (7:3) |
| Flow rate | 1.5ml/min. |
| Detection | UV 230nm |

### Example 3:

According to the same method described in Example 2, an extract was extracted with ethanol from the unheated powdered Job's-tears of production district A. Then, solvent was removed therefrom with a rotary evaporator, and a Job's-tears extract (an extract solution) was obtained. The obtained extract was heat-processed at 150°C for 3 hours in a forced convection constant temperature oven to obtain a Job's-tears-derived antibacterial substance. Using *Bacillus subtilis* as a test bacteria, the obtained heat-processed Job's-tears-derived antibacterial substance was tested according to the same method as in Example 1, thereby determining MIC (minimum inhibitory concentration). The result is shown in Table 1.

### Control example 1:

MIC was measured according to the same method as in Example 1, with the only exception being that the Job's-tears powders used were unheated. The result is shown in Table 1.

### Control example 2:

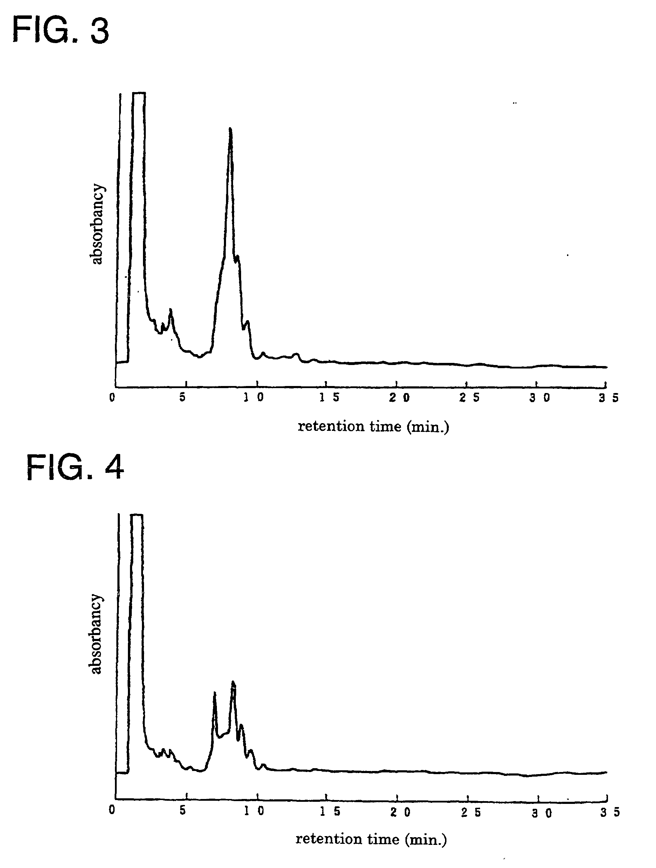

A Job's-tears extract was obtained according to the same method as in Example 2, with the only exception being that the used Job's-tears powders were unheated, and then a chromatogram and MIC were obtained. The chromatogram of the substance of production district A is shown in Figure 3, and that of production district B is shown in Figure 4. Furthermore, each MIC value is shown in Table 1.

### Control example 3:

MIC was measured according to the same method as in Example 3, with the only exception being that the obtained Job's-tears extract was not subjected to heat-processing. The result is shown in Table 1.

**Table 1.**

| | Example 1 | Example 2 | | Example3 | Control 1 | Control 2 | | Control 3 |
|---|---|---|---|---|---|---|---|---|
| | | PD-A | PD-B | | | PD-A | PD-B | |
| MIC | 0.4% | 0.05% | 0.05% | 0.2% | more than 1.6% | 0.8% | more than 1.6% | more than 1.6% |
| PD: production district | | | | | | | | |
| Control: control example | | | | | | | | |
| MIC: minimum inhibitory concentration | | | | | | | | |

### Example 4:

### Fraction of total lipids

150ml of a mixture of methanol and chloroform (1:2) was added to 1g of antibacterial substance of the Job's-tears of production district A obtained in Example 2. Then, while triturating, the mixture was extracted, and filtrated. By the same method stated above, the filtrated residue was extracted while triturating, and filtrated again. After both filtrates were mixed, the total filtrate was moved to a separatory funnel, then a quarter volume of 0.88% potassium chloride solution was added thereto and fully shaken. After that, the mixture was left to divide into 2 layers. The lower chloroform layer was collected, and chloroform was removed therefrom using a rotary evaporator, thereby obtaining 0.49g of total lipids (which is defined as fraction 1) contained in the Job's-tears-derived antibacterial substance of production district A. Moreover, the residue in the above filtration process and the upper water-methanol layer in the above filtrate division process were mixed and concentrated, thereby obtaining the concentrated residue of lipid extract (which is defined as fraction 2).

### Fraction of total lipids by preparative HPLC

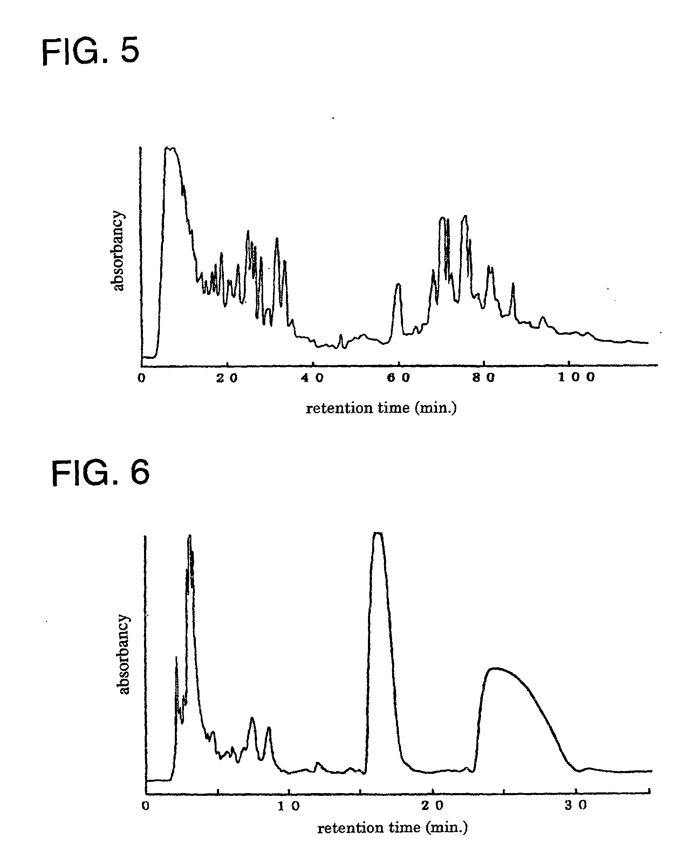

After adding ethanol to the above-obtained total lipids, the mixture was dispersively decomposed and filtrated with a HPLC pretreatment filter, whose pore size was 0.45 *µ* m. The obtained filtrate was subjected to HPLC, and each of the following 3 fractions was fractionated: a fraction at elution time 0 to 20 min. (fraction 3), a fraction at elution time 20 to 40 min. (fraction 4) and a fraction at elution time 40 to 115min. (fraction 5). Then, eluant was removed from each of the obtained solutions corresponding to fractions 3 to 5. The preparative HPLC was carried out under the following conditions, and the obtained chromatogram is shown in Figure 5.

| Conditions on preparative HPLC | |
|---|---|
| Preparative column | Inertsil PREP-ODS (reversed-phase partition system, GL Sciences Inc.) |
| Column size | 20.0mm × 250mm |
| Eluant | Liquid A (acetonitrile and water (7:3)) |
| | Liquid B (acetonitrile) |
| Gradient | 0 to 50 min. A |
| | 50 to 70 min. A→B linear gradient |
| | 70 to 115 min. B |
| Flow rate | 10.0ml/min. |
| Detection | UV 195nm |
| Column temperature | 40° C |

Using *Bacillus subtilis* as a test bacteria, a Job's-tears-derived antibacterial substance obtained in the present examples (fraction 1) and its fractions (fractions 3, 4 and 5), and the above-stated fraction 2 were tested according to the same method as in Example 1, thereby determining MIC (minimum inhibitory concentration). The result is shown in Table 2.

**Table 2.**

| | Example 4 | | | | |
|---|---|---|---|---|---|
| | Fraction 1 | Fraction 2 | Fraction 3 | Fraction 4 | Fraction 5 |
| MIC (%) | 0.005 | more than 0.4 | 0.05 | 0.001 | 0.1 |
| MIC: minimum inhibitory concentration | | | | | |

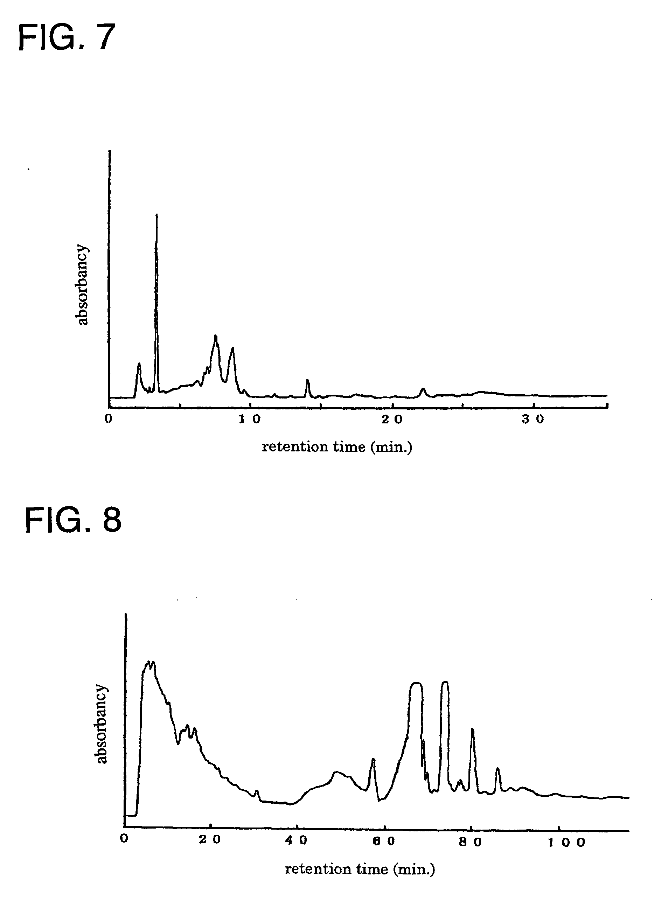

Among 3 fractions obtained by preparative HPLC for total lipids, fraction 4 showed the strongest antibacterial effect. Hence, fraction 4 was analyzed with analytical HPLC under the following conditions. The chromatogram obtained by analytical HPLC for total lipids (fraction 1) is shown in Figure 6, and that obtained by analytical HPLC for total lipids (fraction 4) is shown in Figure 7. An ethanol solution containing linolic acid was analyzed under the same conditions. The retention time of linolic acid was approx. 15 to 16 minutes (Column pressure: 70kgf/cm²).

| Conditions on analytical HPLC | |
|---|---|
| Column | YMC-Pack ODS-AQ (S-5, 120A) (YMC) |
| Column size | 4.6mm × 250mm |
| Eluant | acetonitrile and water (8:2) |
| Flow rate | 1.0ml/min. |
| Detection | UV 195nm |
| Column temperature | 40°C |

### Control example 4:

Total lipids were obtained from the Job's-tears extract of production district A obtained in the above Control example 2, and were subjected to preparative HPLC. The obtained chromatogram is shown in Figure 8.

In addition, MIC (minimum inhibitory concentration) was determined in respect of each of fractions 1 to 5 which was obtained from the Job's-tears extract of production district A. The results are shown in Table 3.

**Table 3.**

| | Control example 4 | | | | |
|---|---|---|---|---|---|
| | Fraction 1 | Fraction 2 | Fraction 3 | Fraction 4 | Fraction 5 |
| MIC (%) | 0.1 | more than 0.4 | 0.1 | 0.05 | 0.1 |
| MIC: minimum inhibitory concentration | | | | | |

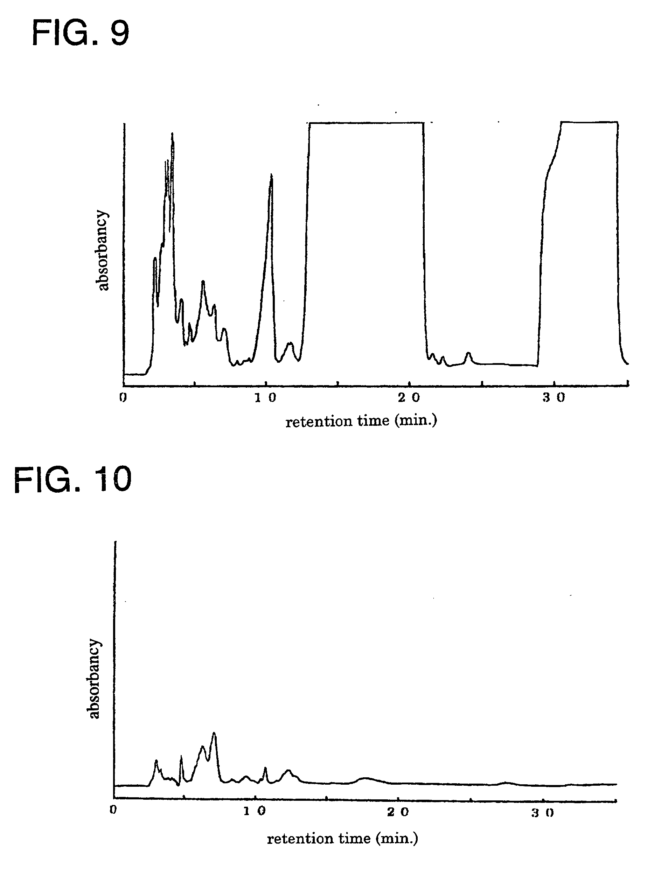

For reference purposes, the chromatogram showing the result of analytical HPLC for total lipids of the Job's-tears extract of production district A, which was obtained in the above Control example 2, is shown in Figure 9, and the chromatogram showing the result of analytical HPLC for lipids corresponding to fraction 4 is shown in Figure 10.

From both the results shown in Tables 2 and 3 and the result of analysis of an antibacterial component contained in a heat-processed Job's-tears extract, it is clear that total lipids contained in a heat-processed Job's-tears extract contribute an antibacterial effect, and that, among the total lipids, lipids corresponding to fraction 4, which were eluted at retention time of approx. 20 to 40 min. in the chromatogram of Figure 5, have the strongest antibacterial effect (in the chromatogram of Figure 6, elution time is approx. 6 to 10 min.)

In contrast, apart from total lipids, other components of a Job's-tears extract do not show any particularly strong antibacterial effect. Furthermore, in comparison to the case where heat-processed Job's-tears is used, the antibacterial effect of lipid components and a component corresponding to fraction 4 obtained from an unheated Job's-tears extract is rather weak.

Accordingly, it can be confirmed that a change occurs to the lipid components of Job's-tears and a component corresponding to fraction 4 having a strong antibacterial effect is generated by heating.

### Example 5:

Both 0.025 weight % of antibacterial substance contained in an Job's-tears extract of production district A obtained in Example 2 and any one of the amino acids, organic acids and organic acid salts, and ethanol, described in Table 4 set forth below, were added to a SCD medium (a triptosoy agar medium) so that the concentration of each additive substance could be set to an established concentration as described in Table 4. Then, an inoculating loop of suspension of *Bacillus subtilis* (approx. 10⁶/ml) was applied thereto, and cultured at 35°C. The existence or nonexistence of viable cells after 18 hours is shown in Table 4.

**Table 4.**

| | | | | | | | |
|---|---|---|---|---|---|---|---|
| The antibacterial effect of the combined use of Job's-tears extract and various additive substances to *Bacillus subtilis* | | | | | | | |

| (additive amount of Job's-tears extract: 0.025%) | | | | | | | |
|---|---|---|---|---|---|---|---|
| | citric acid | sorbic acid | fumaric acid | malic acid | sodium acetate | alanine alamne | ethanol ethanol |
| Additive amount (%) | 0.05 | 0.05 | 0.03 | 0.03 | 2.0 | 5.0 | 2.0 |
| Viable cell | - | - | - | - | - | - | - |
| +: viable cells exist | | | | | | | |
| -: viable cells do not exist | | | | | | | |

### Control example 4:

The antibacterial effect of each additive substance absent the use of the Job's-tears extract in Example 2 in combination, was tested by the same method as in Example 5.
The results are shown in Table 5.

**Table 5.**

| | | | | | | | | |
|---|---|---|---|---|---|---|---|---|
| The antibacterial effect of various additive substances to *Bacillus subtilis* | | | | | | | | |

| | Job'stears extract | citric acid | sorbic acid | fumaric acid | malic acid | sodium acetate | alanine | ethanol |
|---|---|---|---|---|---|---|---|---|
| Additive amount (%) | 0.025 | 0.05 | 0.05 | 0.03 | 0.03 | 2.0 | 5.0 | 2.0 |
| Viable cell | + | + | + | + | + | + | + | + |
| MIC(%) | 0.05 | 0.5 | 0.2 | 0.25 | 0.25 | more than 4.0 | more than 10 | 10.0 |
| +: viable cells exist | | | | | | | | |
| -: viable cells do not exist | | | | | | | | |

As is clear from Tables 4 and 5, which indicate the result of Example 5 and Control example 4 respectively, it is apparent that the combined use of a Job's-tears extract and another substance exhibits effective growth inhibitory activity against *Bacillus subtilis*, even though the concentration of the Job's-tears extract by itself is too low to inhibit cell growth. As is clear from Tables 4 and 5, the extent of improved antibacterial effect by combined use of another substance is tens to hundreds of times lower than MIC determined in the single use of each substance.

It is further confirmed that the combined use of a Job's-tears-derived component that is not heat-processed and various substances described in Table 4 can also provide an improved antibacterial effect.

### Example 6:

Both 0.8 % of Job's-tears extract and any one of amino acids, organic acids or organic acid salts, and ethanol, were added to a SCD medium so that the concentration of each additive substance could be set in an established concentration. Then, an inoculating loop of a suspension of *Escherichia coli* (approx. 10⁶/ml) was applied thereto, and cultured at 35°C. The existence or nonexistence of viable cells after 18 hours is shown in Table 6.

### Control example 5:

The antibacterial effect of each additive substance absent the use of Job's-tears extract in combination was tested by the same method as in Example 6. The results are shown in Table 7.

**Table 6**

| | | | | | | | |
|---|---|---|---|---|---|---|---|
| The antibacterial effect of the combined use of Job's-tears extract and various additive substances to *Escherichia coli* | | | | | | | |

| (additive amount of Job's-tears extract: 0.8%) | | | | | | | |
|---|---|---|---|---|---|---|---|
| | citric acid | sorbic acid | fumaric acid | malic acid | sodium acetate | alanine | ethanol |
| Additive amount (%) | 0.15 | 0.1 | 0.06 | 0.125 | 2.0 | 5.0 | 5.0 |
| Viable cell | - | - | - | - | - | - | - |

**Table 7.**

| | | | | | | | | |
|---|---|---|---|---|---|---|---|---|
| The antibacterial effect of various additive substances to *Escherichia coli* | | | | | | | | |

| | Job'stears extract | citric acid | sorbic acid | fumaric acid | malic acid | sodium acetate | alanine | ethanol |
|---|---|---|---|---|---|---|---|---|
| Additive amount (%) | 0.8 | 0.15 | 0.1 | 0.06 | 0.125 | 2.0 | 5.0 | 5.0 |
| Viable cell | + | + | + | + | + | + | + | + |
| MIC(%) | more than 0.8 | 0.3 | 0.2 | 0.25 | 0.25 | 8.0 | more than 10 | 8.0 |

As is clear from Tables 6 and 7, the combined use of a Job's-tears extract and another substance exhibits effective growth inhibitory activity against *Escherichia coli,* even though the concentration of the Job's-tears extract by itself is too low to inhibit the cell growth. Thus, through the combined use of Job's-tears extract and another substance, the antibacterial spectrum of Job's-tears extract can be extended.

### Example 7:

50g of starch and 100g of water were added to 1kg of commercial cod paste and kneaded. Various additive substances were added and then further kneaded to make the mixed paste uniform. 15g each of the obtained mixed paste blocks was steamed for 15 minutes and then left at room temperature for 15 minutes. After cooling, each block was put into a plastic case and kept at 15°C. The retention period was determined on the basis of smell and appearance of each block. The results are shown in Table 8.

**Table 8.**

| No. | Amount of each additive substance to food (%) | | | Retenrion period at 15°C |
|---|---|---|---|---|
| | Job's-tears extract | sodium acetate | gluconodeltalactone | |
| 1 | 0 | 0 | 0 | 1 |
| 2 | 0.05 | 0 | 0 | 3 |
| 3 | 0 | 0.475 | 0 | 2 |
| 4 | 0 | 0 | 0.375 | 3 |
| 6 | 0.05 | 0.475 | 0.3 | 9 |

### Example 8:

200g of onion, 300g of shrimp paste, 30g of spring onion and condiment were added to 600g of pork, and kneaded. Then, each of the additive substances was added thereto so that an established concentration was obtained. 15g each of the obtained mixed paste was wrapped with a commercial shao-mai coating. After steaming in hot water vapors for 15 minutes, each block was left at room temperature for 15 minutes to cool. After that, each block was put into a plastic case and kept at 15°C. The retention period was determined on the basis of smell and appearance of each block. The results are shown in Table 9. As shown in Table 9, through the combined use of a Job's-tears extract and other substances, the retention period of food can be extended.

**Table 9.**

| No. | Amount of each additive substance to food (%) | | | Retention period at 15°C |
|---|---|---|---|---|
| | Job's-tears extract | sodium acetate | gluconodeltalactone lactone | |
| 1 | 0 | 0 | 0 | 2 |
| 2 | 0.05 | | 0 0 3 | |
| 3 | 0 | 0.475 | 0 | 2 |
| 4 | 0 | 0 | 0.375 | 3 |
| 6 | 0.05 | 0.475 | 0.3 | 9 |

### Industrial Applicability

As is clear from the above results, according to the present invention, a Job's-tears-derived antibacterial component having a strong antibacterial effect can easily be obtained, regardless of the species of Job's-tears used as a material. Furthermore, the present invention provides an antibacterial composition, whose antibacterial effect is more improved than that of the above Job's-tears-derived antibacterial component.

## Claims

1. An antibacterial agent which comprises heat-processed Job's-tears.

2. An antibacterial agent which comprises an extract from heat-processed Job's-tears or a heat-processed product of an extract from Job's-tears.

3. An antibacterial agent which comprises lipid components obtained from, an extract from heat-processed Job's-tears or a heat-processed product of an extract from Job's-tears.

4. The antibacterial agent according to claim 3, wherein said lipid components are simple lipid components.

5. The antibacterial agent according to claim 3 or 4, wherein said lipid components, which are obtained from said extract from heat-processed Job's-tears and/or said product of the heat-processed extract from Job's-tears, are lipid components corresponding to a fraction of preparative high performance liquid chromatography at retention time 20 to 40 min., conducted under the following conditions:
| Conditions on preparative HPLC | |
|---|---|
| Preparative column | Inertsil PREP-ODS (reversed-phase partition system, GL Sciences Inc.) |
| Column size | 20.0mm × 250mm |
| Eluant | Liquid A (acetonitrile and water (7:3)) |
| | Liquid B (acetonitrile) |
| Gradient | 0 to 50 min. A |
| | 50 to 70 min. A→B linear gradient |
| | 70 to 115 min. B |
| Flow rate | 10.0ml/min. |
| Detection | UV 195nm |
| Column temperature | 40°C |

6. An antibacterial agent, which comprises:
at least one Job's-tears-derived component selected from a group which consists of a component consisting of an antibacterial agent described in any one of claims 1 to 5, and another component consisting of an extract or an enzymolysis extract from unheated Job's-tears; and,
at least one component selected from a group which consists of amino acids, amino acid salts, carboxylic acids, carboxylic acid salts, inorganic acids, inorganic acid salts, fatty acid esters, ethanol, protamine and lysozyme.

7. An antibacterial agent, which comprises:
at least one Job's-tears-derived component selected from a group which consists of a component consisting of an antibacterial agent described in any one of claims 1 to 5, and another component consisting of powders or an extract obtained from unheated Job's-tears; and,
at least one component selected from a group consisting of glycine, alanine, glutamic acid, asparatic acid, acetic acid, lactic acid, succinic acid, citric acid, sorbic acid, fumaric acid, malic acid, tartaric acid, gluconic acid, glutaric acid, adipic acid, propionic acid, benzoic acid, cinnamic acid, hydrochloric acid, pyrophosphoric acid, phosphoric acid, polyphosphoric acid, phytic acid or phytic acid salts, gluconodelta-lactone, ethanol, glycerine fatty acid ester, sucrose fatty acid ester, sorbitan fatty acid ester, propylene glycol fatty acid ester, protamine and lysozyme.

8. A method for antibacterial processing of a target which comprises:
using a component contained in an antibacterial agent described in any one of claims 1 to 5; or
using at least one Job's-tears-derived component selected from a group consisting of said component contained in the antibacterial agent, and powders or an extract obtained from unheated Job's-tears, in combination with at least one component selected from a group consisting of amino acids, amino acid salts, carboxylic acids, carboxylic acid salts, inorganic acids, inorganic acid salts, fatty acid esters, ethanol, protamine and lysozyme.

9. A method for producing an antibacterial component for an antibacterial agent, which comprises heat-processing Job's-tears and/or an extract from Job's-tears at 50 to 250°C.

10. A method for producing an antibacterial component for an antibacterial agent, which comprises heat-processing Job's-tears and/or an extract from Job's-tears at 50 to 250°C, and separating lipid components from said heat-processed Job's-tears and/or extract from Job's-tears.

11. A method for producing an antibacterial component for an antibacterial agent, which comprises obtaining lipid components from a heat-processed powdered Job's-tears or a product of an extract from unheated Job's-tears, wherein said lipid components correspond to a fraction of preparative high performance liquid chromatography at retention time 20 to 40 min., conducted under the following conditions:
| Conditions on preparative HPLC | |
|---|---|
| Preparative column | Inertsil PREP-ODS (reversed-phase partition system, GL Sciences Inc.) |
| Column size | 20.0mm × 250mm |
| Eluant | Liquid A (acetonitrile and water (7:3)) |
| | Liquid B (acetonitrile) |
| Gradient | 0 to 50 min. A |
| | 50 to 70 min. A→B linear gradient |
| | 70 to 115 min. B |
| Flow rate | 10.0ml/min. |
| Detection | UV 195nm |
| Column temperature | 40°C |
